# EUROPEAN PATENT APPLICATION

(11) **EP 3 170 393 A1**
(43) Date of publication of application: **24.05.2017**
(21) Application number: 15464007.2
(22) Date of filing: 24.11.2015
(51) Int. Cl.: A01N 25/04, A01N 61/00, A23J 1/10, C12P 21/06

(54) **COLLAGEN POLYDISPERSIONS FOR THE TREATMENT OF CEREAL SEEDS AND PROCESS THEREOF**

(30) Priority: 18.11.2015 RO 201500859
(71) Applicant: Institutul National de Cercetare-Dezvoltare Pentru Textile si Pielarie (INCDTP) Sucursala Institul de Cercetare Pielarie Incaltaminte (ICPI), 031215 Bucuresti (RO)
(72) Inventor: Niculescu, Mihaela-Doina, Bucuresti (RO); Gaidau, Carmen Cornelia, Bucuresti (RO); Epure, Doru-Gabriel, Judetul Olt (RO); Gîdea, Mihai, Judetul Teleorman (RO); Stepan, Emil, Bucuresti (RO)

(57) **Abstract**

The invention relates to a product based on collagen polydispersions used for cereal seed treatment in order to stimulate growth, reduce the intake of pesticides and the process of manufacturing thereof.

Extraction of collagen polydispersions from leather waste occurs in two phases carried out through a compact process in the first phase through alkaline hydrolysis of chrome tanned leather waste, with 8-12% calcium oxide, or thermal hydrolysis of rawhide waste, at a solid/liquid ratio of 1/4, at a temperature of 70.....85°C, stirring for a period of 3.....5 hours and static until the next day without thermal input, followed by enzymatic hydrolysis with enzymatic preparation Alcalase 2.4L in concentration of 2.5.....4.5 enzyme units/total nitrogen gram, at a temperature of 63°C ± 2°C continuously stirring for 3.....5 hours for tanned leather waste, and static until the next day without thermal input in the case of extraction from rawhide waste. This process enables protein mass recovery in a proportion of 85.....95% and cleavage of peptide chains, yielding collagen polydispersions containing free amino acids, with average molecular weight of 4000-6000 Da for systemic cereal seed treatment, with effects on stimulating germination and increasing resistance to insect and fungal attacks.

## Description

The invention relates to a product based on collagen polydispersions used for cereal seed treatment in order to stimulate growth, reduce the intake of pesticides and the process of manufacturing thereof.

Products based on protein hydrolysates of plant and animal resources are already known for environmentally friendly foliar and root fertilisation of plants. Foliar fertilizers containing synthetic amino acid or collagen hydrolysates (RO Patent 123026 B1) that stimulate plant growth and nutrition are also known.

The problem solved by this patent is to make a collagen polydispersion for cereal seed treatment in order to increase nutritional and health status of seeds and reduce the amount of pesticides used to treat thereof. Ecological and economic effects of applying the new product are high given the demand for organic products, soil conservation and reducing the amount of pesticides such as imidacloprid and tebuconazole.

Methods are known for treating cereal seed with chitosan (US Patent 4886541 A) to increase the production of plants, root thickness and stalk strength. The method of treating cereal seed with polysaccharides including pectin in film-forming mixtures against fungi and insects is also known (US Patent 20130225403 A1). There are also patented complex compositions based on condensed hydrocarbons, lignins, and tannins (EP Patent 2473034 A2) with effects on at least one feature of germination, emergence, root development, vigor and growth of seedlings, decreased mortality, the production of chlorophyll, resistance to cold, drought, nutrient absorption, compared to untreated seeds.

The disadvantages of these methods of treatment consist in the complexity of the materials used and of the composition, incomplete information on their testing level and reproducibility of results.

This invention solves the problem of treating cereal seeds by providing a single product with nutritional properties given by the content of easily assimilated and natural, free amino acids which provide protection by forming a polymeric collagen film on the surface of seeds able to adsorb water due to their amphoteric character and to release amino acids over time from the structure of oligopeptides and polypeptides.

Laboratory and experimental field testing of winter wheat seeds treated with collagen polydispersions demonstrated the following advantages:
- Increasing gibberellic acid, growth hormone precursor, by 30-40% after 18 and 24 hours from emergence;
- Increasing emergence density by 6-12%;
- Increasing biomass by 5-10%;
- Reduction in abnormal seeds by 4-8%;
- The possibility of reducing the concentration of pesticides by 50-70%;
- Stimulation of plant growth in adverse soil conditions, at extreme pH values (6.0 and 8.9).

The main features of patented collagen polydispersions are: 4000-6000Da molecular weight, composition formed from a population with 60% share of 1-10 nm particle sizes and two populations with 14% and 26% share of 100-1000 nm and 1000-5000 nm, respectively. The polydispersity index is 0.6. The concentration of free amino acids is 6-10% and contains methionine, glutamine, alanine, glycine, leucine, etc., with a role in plant metabolism and the mechanisms of adaptation to adverse climate conditions.

The process for making the collagen polydispersion, also called collagen hydrolysate, for the treatment of seeds, allows to obtain components with average molecular weight within controlled limits from semi-processed cattle leather by-products, by means of thermo-enzymatic catalysis.

At present, there is a wide array of biopolymers - materials based on proteins extracted from collagen and materials with collagen matrix, used mainly in the food, medical, pharmaceutical and cosmetics industries, for whose implementation primary collagen resources are used [1, 2]. An alternative to primary protein resources are secondary resources such as rawhide waste from the leather industry, which is not used, although they may lead to similar performance to that of major resources and exclude contamination risks because they have already undergone severe enough chemical processing to destroy bacteria and viruses. Also, for the extraction of proteins used in agriculture, secondary collagen resources, such as by-products from the leather processing industry, represent a viable alternative. Most of the research in recent years has been focusing on extracting collagen from leather waste [3, 4] and using these extracts in crop fertilization [5, 6], as an alternative for synthetic amino acids used in the formulas of fertilizers [7, 8]. Other recent research is focused on the exploitation of bio-active properties of these collagen extracts in treating seeds to increase their germination potential and protection against pests [9, 10]. There is recent research that studies the possibility of using collagen recovered from the leather industry for remediation of agricultural soil [11], aiming at using collagen from rawhide waste. However, there is another collagen resource consisting of tanned leather waste.

Compared to research so far, focused on the recovery of collagen from leather without chromium content, the invention proposes the recovery of collagen, including from leather waste tanned with chromium (III), a resource avoided systematically due to the psychological impact generated by the term "chrome", generally associated with the terms "heavy metal", "toxic", and "carcinogenic", without taking into account that in the last 20 years the leather industry has been complying with good practices wherein hexavalent chromium which generates extreme toxic effects is not used and discharged. Previous research [6, 12] demonstrated that various combinations of processes for extracting collagen from such waste/by-products may be highly effective in separating chromium, so that its content in collagen polydispersions would be limited to an admissible level (of the order of 10² ppb) in relation to applications it addresses, below the values (of the order of 10² ppm) reported in other works [13].

Procedures are known by means of which chrome-tanned leather waste is hydrolysed in alkaline medium (US Patent 4483829, US Patent 4100154), or through enzymatic preparations (US Patent 5094946, RO Patent 126673 A2) to separate proteins from mineral compounds for the purpose of using them in nutritional supplement formulas for animal feeds, as fertilizer, and in the composition of cosmetic preparations. Other more recent processes propose bone meal (CN Patent 103243143-A) or pigskin (CN Patent 104673863-A) as raw material for collagen hydrolysate, from which collagen is extracted at high pressure by alkaline enzymatic hydrolysis. There is also a process that uses carbonated water (JP Patent 2013245198-A) to extract collagen for applications in cosmetics, and gelatine used as a dispersing agent for electrical charge in electronic devices.

These processes have the following disadvantages:
- leather waste used as raw material requires prior mechanical fragmentation operations;
- alkaline hydrolysis temperature is above 90°C, close to the reflux temperature;
- chromium content of protein hydrolysates are of the order of 10² ppm at pH<11;
- protein hydrolysates used as fertilizers have a low free amino acid content;
- processes are not designed in order to obtain collagen hydrolysates with low average molecular weights guaranteeing a certain amount of essential free amino acids, recognized for their important contribution to plant metabolism, especially under stress conditions;
- processes are conducted in several stages, requiring intermediate filtration and final conditioning, which involves additional labour and energy;
- bone meal is extracted at high pressures;
- using pigskin as raw material involves additional operations to remove fatty substances;
- supplying carbonated water is a tedious approach in the specific conditions of tanneries and the extraction process requires a longer duration than other processes.

The technical problem solved by the invention consists in obtaining collagen extracts with high polydispersity, made up of polypeptides, oligopeptides and free amino acids, including essential amino acids, by developing a process of compact thermo-enzymatic hydrolysis of semi-processed leather waste, tanned or untanned. In the first phase of the process, thermal distortion of collagen occurs and gelatine is extracted, and in the second phase, polypeptide fragmentation occurs through enzyme-catalyzed hydrolysis and the average molecular weight is reduced, when amino acids are released, including essential amino acids.

Both phases of the process take place in closed reaction vessels, equipped with an insulated thermostatic stirring system and a heating-cooling jacket.

The following can be used as raw materials: residual chrome tanned leather fragments with a volatile matter content of 50-60% and characterized by a dermal substance content of min. 85% (relative to the volatile matter-free product), chromium expressed as chromium oxide, min. 3.5% (relative to the volatile matter-free product), and the rest consisting of salts from the preparation of the collagen substrate for tanning; semi-processed rawhide, called pelt, with the following characteristics: 65-75% volatile matter, dermal substance min. 95% (relative to the volatile matter-free product), the rest consisting of salts from the unhairing and deliming phase for preparation of the collagen substrate.

Fragments of leather waste (as such if tanned or ground if untanned) analyzed in terms of their volatile matter content, total nitrogen, dermal substance, possibly chromium, are dispersed in water in a solid/liquid ratio of 1/4 and subjected to a thermal regime under stirring. If the raw material consists of fragments of chrome tanned leather, this phase takes place in the presence of calcium oxide, which serves to coagulate sulphate ions in the form of calcium sulphate and to precipitate chromium released as chromium hydroxide, the coprecipitation of the two compounds contributing to the efficient separation of chromium hydroxide from the dispersion resulting from hydrolysis. If the raw material is pelt waste, calcium oxide dosing is not required. Gelatine extraction is performed under continuous stirring, at the temperature of 70.....85°C, for a period of 3.....5 hours. For the second phase of the process, i.e. hydrolysis, the pH is checked and adjusted with sulphuric acid diluted in a ratio of 1:10, up to a value in the range of 8.5.......9.0, and under continuous stirring, the liquid enzyme preparation Alcalase 2.4L is dosed in an amount equivalent to a ratio of 2.5.....4.5 enzyme units/total nitrogen gram, maintained at 63°C ± 2°C over a period of 3.....5 hours under continuous stirring, then heated to 80.....90°C to deactivate the enzyme.

The warm dispersion is vacuum filtered on a Nutsche filter using a material with 2-4 µm porosity. The filtrate consisting of collagen hydrolysate with an average molecular weight of 4000-6000 Daltons is analyzed to determine total nitrogen and amino nitrogen and to establish pH. Amino nitrogen content provides clues on the level of average molecular weight of the obtained polydispersion. Peptide chain cleavage occurring during enzymatic hydrolysis in the second phase of the process diversifies the content of amino acids, including essential amino acids, in the collagen polydispersion for applications in systemic treatment of cereal seed.

The process for making collagen polydispersions from leather industry waste, according to the invention, eliminates the above mentioned disadvantages in that:
- it is versatile and can be used with minor adaptations for both untanned and tanned leather waste;
- it is compact and requires no intermediate filtering;
- it uses residual leather fragments resulting after the shaving operation of leather tanned with basic chromium salts, humidity 55.....60%, with a content of 85.....90% dermal substance (relative to the volatile-matter free product) and 4.0.....5.0% chromium oxide (relative to the volatile-matter free product), without requiring mechanical fragmentation operations;
- it can be adapted for tanned leather waste; fragmentation is simply done by using grinding machines similar to those used in the food industry;
- the first phase consists in thermal extraction at a solid/liquid ratio of 1/4, using 8-12% calcium oxide at 70.....85°C over a period of 3.....5 hours under continuous stirring, if using chrome tanned leather waste as raw material, or without calcium oxide, in the same conditions, if rawhide waste is used as raw material.
- the second phase consists in enzymatic hydrolysis, using Alcalase 2.4L (or similar products) at the temperature of 63°C ± 2°C for 3.....5 hours, under continuous stirring, ensuring polypeptide fragmentation and reduction of average molecular weight of the collagen extract, with the effect of amino acid content diversification, including essential amino acids;
- in this way it ensures the protein component extraction with 85.....95% yields;
- collagen polydispersions obtained by this process allow film coating of cereal seeds that provides both immediate intake of free amino acids that stimulate germination, and a gradual intake of amino acids that nourish the seedlings, giving them an increased resistance which reduces the amount of conventional synthetic pesticides in treatments against insects and fungi.

The following advantages are obtained from applying the invention:
- high yield recovery of protein component from semi-processed leather waste with all the favourable consequences of their disposal, namely reducing the space needed for storage, reducing transportation costs and emissions, compliance with environmental legislation in force, conservation of natural resources through advanced recovery;
- exploitation of an alternative resource of levorotatory amino acids, assimilated by plants without additional energy consumption in order to increase production in agriculture, reducing the consumption of synthetic amino acids which have the disadvantage of containing enantiomers, an issue still unresolved by research in the last few years [14];

- waste from an industrial sector is converted into an useful by-product to increase eco-efficiency in bio-economy (by partial or total replacement of synthetic amino acids, which require greater direct financial resources for production and indirect resources for environmental protection);
- it encourages the development of materials and practices supporting organic agriculture (organic, biological) that prohibits the use of any substance obtained by chemical synthesis processes.
- the compact process for obtaining collagen extracts in the form of hydrolysates according to the invention is simple, effective, economical, reproducible;
- the range of collagen hydrolysates can be varied as needed;
- the use of collagen polydispersions in cereal seed treatment provides an alternative for accelerated germination, increase of plant resistance and increase of agricultural production with a lower consumption of synthetic substances used for treatments of compensating hydronutritional deficiencies and for pest and fungi control, etc.;
The following are two embodiments of the invention:

### Embodiment 1

Residual fragments of chrome leather resulting after the shaving operation of leather tanned with basic chromium salts analysed to determine chromium content, total nitrogen, and dermal substance, are dispersed in a water volume equivalent to a solid/liquid ratio of 1/4 in a reaction vessel equipped with heating-cooling jacket and insulation, stirring system and automatic temperature control, are treated with 8-12% calcium oxide (amount equivalent to complete precipitation of chromium content). The reaction mass is heated to 70.....85°C and continuously stirred at this temperature for 3.....5 hours, then left to rest until the next day, while the hydrolytic process continues due to inertia and to the temperature remaining high for many hours after decoupling heating source due to the insulating layer of the reaction vessel. Meanwhile leather fragments are disaggregated, chromium is precipitated as chromium hydroxide and the protein component passes into the solution in proportion of 85.....95%. The next day the reaction mass is stirred, the pH of the collagen hydrolysate is checked and if necessary adjusted with 1:10 diluted sulfuric acid, until reaching a value in the range 8.5.....9.0, heated at 63°C ± 2°C under continuous stirring and enzymatic preparation Alcalase 2.4L is dosed in an amount equivalent to a ratio of 2.5.....4.5 enzyme units/total nitrogen gram, maintained at of 63°C ± 2°C for 3.....5 hours, stirred continuously, then heated to 85.....90°C to deactivate the enzyme. The dispersion is discharged from the vessel, allowed to settle until the next day, then the decanted product is filtered using a vacuum filter (Nutsche filter) and the filtrate is analyzed to determine total nitrogen and amino nitrogen, which will indicate an average molecular weight of 4000-6000 Daltons. Chromium content is then checked.

### Embodiment 2

Residual fragments of rawhide analysed for the determination of total nitrogen and dermal substance are ground and then dispersed in a water volume equivalent to a solid/liquid ratio of 1/4 in a reaction vessel equipped with heating and cooling jacket and insulation, stirring system and automatic temperature control, heated to 70.....85°C and stirred continuously at that temperature for 3.....5 hours, then the pH is checked, and adjusted, if necessary, with sulphuric acid (diluted 1:10), so that the pH of the dispersion would reach a value in the range of 8.5.....9.5, and under continuous stirring, the reaction mass is cooled to 63°C±2°C, then enzymatic preparation Alcalase 2.4L is dosed in an amount equivalent to a ratio of 2.5.....4.5 enzyme units/total nitrogen gram, maintained at 63°C ± 2°C for 3.....5 hours, then static until the next day, during which the hydrolytic process continues due to inertia and to the temperature remaining high for many hours after decoupling the heating source due the insulation of the reaction vessel. The next day it is stirred for 15 min. and heated under continuous stirring, 85.....90°C to deactivate the enzyme. The warm dispersion is vacuum filtered (Nutsche filter) and the filtrate is analyzed to determine total nitrogen and amino nitrogen, indicating an average molecular weight of 4000-6000 Daltons.

### REFERENCES

1. Santos, M. H., et al., Materials Science & Engineering C-Materials For Biological Applications, 2013, 33(2), pp. 790-800;
2. Jayathilakan, K., et al., Journal Of Food Science And Technology-Mysore, 2012, 49(3), pp. 278-293;
3. XXX, Collagen Peptide and Gelatin Market, Global Industry Analisis, Size, Share, Growth Trend and Forecast, 2014-2020, Transparency Market Research;
4. Jian, S., Wenyi, T., Wuyong, Ch., Ultrasound-Accelerated Enzymatic Hydrolysis of Solid Leather Waste, J. Cleaner Production, 16(5), 2008, pp. 591-597;
5. Zainescu, A. G., Constantinescu, R., Voicu, P., et al., Biopolymers from protein wastes used in industry and agriculture, Industria Textila, 2011, 62(1), pp. 34-37;
6. Lacatus, V., Ionita, A., Gaidau, C., Niculescu, M., Popescu, M., Acsinte, D., Filipescu, L., Field test for foliar nutritive products formulated with the leather protein hydrolysates,Ist International Leather Engineering Symposium, Izmir, Turkey, 2009,
7. C. Gaidau, M. Niculescu, E. Stepan, D.Taloi, Laurentiu Filipescu - Additives and Advanced Biomaterials Obtained from Leather Industry By-products, Rev. Chim.-Bucharest, 60(5), 2009, pp. 501-507;
8. Chitu, E., Lacatus, V., Gaidau, C., et al., Emulsied Foliar Nutritive Fluid Effects on the Germination Rates and Leaves Nitrate Accumulation in Plants, Rev. Chem, Bucharest, 2010, 61(11), pp. 1080-1086;
9. Light, M. E., Burger, B. V., Van Staden, J., Formation of a seed germination promoter from carbohydrates and amino acids,J. Agric. Food Chem. 2005, 53, pp. 5936-5942
10. Gaidau, C., Niculescu, M., Stepan, E., Epure, D.-G., Gidea, M., New products based on collagen hydrolysates for cereal seeds treatment and sustainable agriculture, 2nd Biotechnology World Congress, 18-21 feb. 2013, Dubai, UAE,
11. Lomate, P.R., Hivrale, V.K., Changes and induction of aminopeptidase activities in response to pathogen infection during germination of pigeonpea (Cajanas cajan) seeds Journal of Plant Physiology, 2011, 168(15), pp.1735-1742;
12. Zainescu, G., Voicu, P., Gherghina, A., et al., Application of tannery organic wastes in degraded soils remediation, 14th International Biotechnology Symposium and Exhibition (IBS-2008), 2010, Rimini, Italy, Journal of Biotechnology, 2010, 150(1), pp. 290-290;
13. Niculescu, M., Bajenaru, S., Gaidau, C., Simion, D., Filipescu, L., Extraction of the Protein Components as Amino-Acids Hydrolysates from Chrome Leather Wastes Through Hydrolytic Processes, Rev. Chim.-Bucharest, 60(10), 2009, pp. 1070-1078;
14. Kolomaznik, K., et a., Enzimatic Digestion of Chrome Tanned Solid Wasts, 1st International Fur Congress, Kastoria-Greece, 1998, pp. 134-146;
15. Jiang, L., Dziedzic P., Spacil, Z., Zhao, G.L., Nilsson, L., Ilag, 1., Cordova, A., Abiotic synthesis of amino acids and self-crystallization under prebiotic conditions, Scientific Reports, 4:6769, www.nature.com, 2014.

## Claims

1. Collagen polydispersion for the treatment of cereal seed, **characterized in that** it is a mixture of polypeptides, oligopeptides and amino acids with an average molecular weight of 4000-6000Da, containing 60% particles of 1-10 nm size, 26% particles of 1000-5000 nm size, polydispersion index of 0.6, free amino acids concentration of 6-12%, with effects in stimulating and protecting cereal plants and seeds.

2. A process for the extraction of collagen polydispersions, according to claim 1, **characterized in that** it uses residual fragments of leather tanned with basic chromium salts, moisture content 55.....60%, dermal substance content of 85.....90% (relative to the volatile matter-free product) and 4.0.....5.0% chromium oxide (relative to the volatile matter-free product), or uses rawhide waste, with humidity 55.....60% and a dermal substance content of 85.....90% (relative to the volatile matter-free product).

3. A process according to claim 2, **characterized in that** compact thermo-chemical hydrolytical processes are used for the extraction of collagen polydispersions from residual fragments of leather tanned with basic chromium salts, consisting in the first phase in hydrolysis at a solid/liquid ratio of 1/4, with 8-12% calcium oxide at a temperature of 70.....85°C, for 3.....5 hours, continuously stirring, followed in the second phase by enzymatic hydrolysis with Alcalase 2.4L in concentrations of 2.5.....4.5 enzyme units/total nitrogen gram, at 63°C ± 2°C, stirring continuously for 3.....5 hours, then static without thermal input, until the next day, ensuring protein component extraction yields of 85.....95%, in the form of collagen polydispersions with average molecular weight of 4000-6000 Da and containing free amino acids, oligopeptides and polypeptides for the treatment of cereal seeds.

4. The process according to claims 2 and 3, **characterized in that,** in order to extract collagen polydispersions from rawhide waste, thermal processing is used in the first phase, heating the hide dispersion with a solid/liquid ratio of 1/4 up to 70.....85°C for 3.....5 hours, stirring continuously, followed by a second phase, consisting of enzymatic hydrolysis, with Alcalase 2.4L in a concentration of 2.5.....4.5 enzyme units/ total nitrogen gram, at 63°C ± 2°C, stirring continuously for 3.....5 hours, then static without thermal input, until the next day, ensuring protein component extraction yields of 85.....95%, in the form of collagen polydispersions with an average molecular weight of 4000-6000 Da and containing free amino acids, oligopeptides and polypeptides, for the treatment of cereal seeds.
